# EUROPEAN PATENT SPECIFICATION

(11) **EP 1 055 428 B1**
(45) Date of publication and mention of the grant of the patent: **17.11.2004**
(21) Application number: 00110282.1
(22) Date of filing: 23.05.2000
(51) Int. Cl.: A61K 35/78, A61M 15/00, A61P 3/10

(54) **Liquid composition to be vaporized for inhibiting increase in blood sugar lever and vaporizer for the same**
Flüssige Zubereitung zum Verdampfen gegen Erhöhung des Blutzuckerspiegels und Verdampfer für dieselbe
Composition liquide à vaporiser pour inhiber l'augmentation du taux de glycémie sanguine et vaporisateur à cet effet

(30) Priority: 25.05.1999 JP 14454499
(43) Date of publication of application: 29.11.2000
(73) Proprietor: USE Techno Corporation, Fukuchiyama-shi, Kyoto 620-0055 (JP)
(72) Inventor: Matsuyama, Futoshi, c/o Use-Techno Corporation, Fukuchiyama-shi, Kyoto 620-0848 (JP)
(74) Representative: Albrecht, Thomas, Dr.

(56) References cited:
- DATABASE BIOSIS [Online] BIOSCIENCES INFORMATION SERVICE, PHILADELPHIA, PA, US; 1990 MISHRA Y ET AL: "HYPOGLYCEMIC ACTIVITY OF LEAVES OF LAGERSTROEMIA-SPECIOSA L PERS" Database accession no. PREV199191019809 XP002146320 & INDIAN JOURNAL OF PHARMACOLOGY, vol. 22, no. 3, 1990, pages 174-176, ISSN: 0253-7613
- DATABASE BIOSIS [Online] BIOSCIENCES INFORMATION SERVICE, PHILADELPHIA, PA, US; 1996 KAKUDA TAKAMI ET AL: "Hypoglycemic effect of extracts from Lagerstroemia speciosa L. leaves in genetically diabetic KK-A-Y mice." Database accession no. PREV199698796004 XP002146321 & BIOSCIENCE BIOTECHNOLOGY AND BIOCHEMISTRY, vol. 60, no. 2, 1996, pages 204-208, ISSN: 0916-8451
- PATENT ABSTRACTS OF JAPAN vol. 018, no. 122 (C-1173), 28 February 1994 (1994-02-28) & JP 05 310587 A (ITOUEN:KK), 22 November 1993 (1993-11-22)
- PATENT ABSTRACTS OF JAPAN vol. 1995, no. 11, 26 December 1995 (1995-12-26) & JP 07 228539 A (ITOUEN:KK), 29 August 1995 (1995-08-29)

## Description

### Detailed Description of the Invention

### Field of the Invention

The present invention relates to a liquid composition to be vaporized for inhibiting an increase in the blood sugar level of a human being. More specifically, it relates to a liquid composition to be vaporized for inhibiting an increase in blood sugar level, which contains components extracted from Lagerstroemia Speciosa, Linn. or Pers. as active ingredients and has a specific content of corosolic acid and to use thereof.

The present invention relates, specifically, to a composition which is used to inhibit an increase in or lowering the blood sugar level of a human being by vaporizing its active ingredients into the air and causing the active ingredients to be absorbed from the mucous membranes of the throat and the nasal cavity and to a vaporizer for vaporizing the active ingredients into the air.

### Prior Art

Lagerstroemia Speciosa, Linn. or Pers. comes under the Loosestrife Family of Myrtales and is generally called "Queen's Crape Myrtle" as well, and it occurs widely in south east Asian areas including the Philippines, India, Malaysia, southern China and Australia. In the Philippines in particular, dry leaves and flowers of Lagerstroemia Speciosa, Linn. or Pers. are decocted and taken as a drink. This drink is also well known as a folk medicine against diabetes.

Paying attention to leaves of Lagerstroemia Speciosa, Linn. or Pers., an extract thereof has been analyzed to obtain some components. It has been accordingly reported that corosolic acid is contained as one of the components and that the corosolic acid has been studied for its activity by using Ehrlich Ascites Tumour Cells to show that it is a substance which activates the mobility of grape sugar (Chem. Pharm. Bull. 41(12) 2129-2131 (1993)).

The above report is concerned with the results of in vitro experiments and merely suggests the results of a first-stage discrimination test on the anti-diabetes activity of corosolic acid.

JP-A 5-310587 discloses an anti-diabetes preparation containing, as an ingredient, a concentrated dry substance (Lagerstroemia Speciosa, Linn. or Pers. powder extract) extracted from leaves of Lagerstroemia Speciosa, Linn. or Pers. in hot water or an organic solvent. The above preparation is an easily prepared and high-safety anti-diabetes preparation produced by taking out a water-soluble fraction and a lipid-soluble fraction from an extract of leaves of Lagerstroemia Speciosa, Linn. or Pers. and preparing a powder extract from the fraction. The above publication discloses a preferred embodiment in which the powder extract is diluted, e.g., to a concentration of 2 % and taken as a drink, and the anti-diabetes activity thereof is confirmed by an animal experiment using mice diseased with diabetes.

It has been reported that an extract of leaves of Lagerstroemia Speciosa, Linn. or Pers. has the effect of inhibiting an increase in the blood sugar level and is safe according to a clinical test conducted on 24 people (the Health & Medical Department of the Health & Medical Center of Tokyo Jikeikai Medical University, Prof. Yoshio Ikeda et al., Japanese Pharmacology & Therapeutics, Vol. 27, No. 5 published on May 20, 1999). It has also been reported in this thesis that the extract's function of improving sugar transfer activity and insulin resistance has been verified.

As already described, dry leaves of Lagerstroemia Speciosa, Linn. or Pers. have been used as having an effect on the therapy of diabetes in folk medicine. However, it is not clearly known what component(s) of the leaves of Lagerstroemia Speciosa, Linn. or Pers. has/have human anti-diabetes activity. It is known that corosolic acid is contained as one component while the activity thereof is known merely from the result of a study on the function of activating the mobility of grape sugar in in vitro experiments using cells.

Further, there has been no specific clinical information on what component(s) of the extract of leaves of Lagerstroemia Speciosa, Linn. or Pers. has/have activity in the therapy of human diabetes. Moreover, there has been found no information on the relationship between component(s) of the extract of leaves of Lagerstroemia Speciosa, Linn. or Pers. and an increase in human blood sugar level.

The present inventor has therefore studied the relationship between component(s) of the extract of leaves of Lagerstroemia Speciosa, Linn. or Pers. and an increase or inhibition of an increase in human blood sugar level on the basis of clinical tests. When a composition which was a concentrated extract of leaves of Lagerstroemia Speciosa, Linn. or Pers. and which had a specific content of corosolic acid was administered to mild-case diabetes patients who had a fasting blood sugar level slightly higher than approximately 110 mg/dl and who were insulin-non-dependent, it was found that an increase in blood sugar level was inhibited and that the blood sugar levels decreased on average.

According to studies conducted by the present inventor, it has also been found that the composition which has a specific content of the corosolic acid can be obtained by extracting, concentrating and drying leaves of Lagerstroemia Speciosa, Linn. or Pers., under specific conditions.

### Means for Solving the Problem

The present inventor has proposed a composition for inhibiting an increase in or lowering a blood sugar level, which comprises as a main component a concentrate of a hot water or alcohol extract of leaves of Lagerstroemia Speciosa, Linn. or Pers. and has a corosolic acid content of 0.1 to 15 mg per 100 mg of the concentrate.

The above composition proposed by the present inventor is orally administered in the form of a tablet, powder or granular preparation to a patient who has a slightly higher blood sugar level than a normal level or who is expected to suffer an increase in blood sugar level (to be referred to as "quasi-patient" hereinafter). Therefore, the quasi-patient must take the composition several times at predetermined times every day to maintain the effect of the composition. This is very troublesome for the quasi-patient in his/her daily life.

Meanwhile, the present inventor has conducted further studies on the function and mechanism of a concentrated extract of leaves of Lagerstroemia Speciosa, Linn. or Pers. for inhibiting an increase in or lowering a blood sugar level as well as clinical tests using rats.

Surprisingly, it has been found that when the concentrated extract of leaves of Lagerstroemia Speciosa, Linn. or Pers. is vaporized and scattered into the air by heating or nebulizing a solution thereof to administer its volatile components from the mucous membranes of the throat and the nasal cavity through the respiratory passage, it develops the effect of inhibiting an increase in or lowering a blood sugar level. This effect obtained by administration through the respiratory passage is excellent even when an extremely small amount of the concentrate is administered and the present inventor presumes that this effect completely differs from the effect obtained by oral administration previously proposed in function and mechanism.

According to the present invention, there are provided the following inventions.
(I) An aqueous liquid composition to be vaporized for inhibiting an increase in blood sugar level, prepared by dissolving or dispersing a concentrate of a hot water extract or alcohol extract of leaves of Lagerstroemia Speciosa, Linn. or Pers. as an active ingredient in an aqueous medium.
(II) A vaporizer for an aqueous liquid composition for inhibiting an increase in blood sugar level, which comprises (1) a container for containing the above aqueous liquid composition (I) and (2) a porous material which is installed in the container for sucking up the aqueous liquid composition and vaporizing an active ingredient by heating at a top end portion thereof and which extends above the container from a bottom portion of the container.
(III) A vaporizer for an aqueous liquid composition for inhibiting an increase in blood sugar level, which comprises (i) the vaporizer of (II) and (ii) a heater for heating a top end portion of a porous material at an upper portion of the vaporizer.
(IV) A vaporizer for an aqueous liquid composition for inhibiting an increase in blood sugar level, which comprises (1) a container (I) for containing an aqueous liquid composition prepared by dissolving or dispersing an concentrate of a hot water extract or alcohol extract of leaves of Lagerstroemia Speciosa, Linn. or Pers. and (2) an ultrasonic wave generating element installed under the container (I) for nebulizing the aqueous liquid composition in the container (I) and scattering it into the air.
(V) A vaporizer for an aqueous liquid composition for inhibiting an increase in blood sugar level, which comprises:
   (1) a container (I) for containing an aqueous liquid composition prepared by dissolving or dispersing an concentrate of a hot water extract or alcohol extract of leaves of Lagerstroemia Speciosa, Linn. or Pers.,
   (2) a container (II) for containing water, and
   (3) an ultrasonic wave generating element installed under the container (II) for nebulizing the aqueous liquid composition in the container (I) and water in the container (II) and scattering them into the air.

The present invention will be described in more detail hereinafter.

Corosolic acid is one of triterpenoids having the following structural formula.

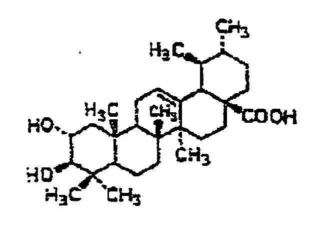

It is considered that the activity of the concentrate of the present invention in inhibiting an increase in or lowering a human blood sugar level is caused by the interaction of corosolic acid in the concentrate and other extracted components of leaves of Lagerstroemia Speciosa, Linn. or Pers.

The leaves of Lagerstroemia Speciosa, Linn. or Pers. used as a raw material for the concentrate used in the formation of the composition of the present invention refer to green leaves of Lagerstroemia Speciosa, Linn. or Pers. which occurs in the Philippines or some other areas or a dry product prepared by drying the same. The green leaves may be dried by leaving them in atmosphere, by air-drying or by forcible drying. Preferably, drying is carried out by so-called toasted-drying until the leaves have a water content of 20 % by weight or less, preferably 10 % by weight or less, for preventing the growth of microorganisms and attaining storage stability.

Dry leaves of Lagerstroemia Speciosa, Linn. or Pers. may be extracted as they are, while it is desirable to pulverize the dry leaves or cut them into pieces before the extraction.

The method and conditions of extracting dry leaves of Lagerstroemia Speciosa, Linn. or Pers. in hot water or an alcohol and concentrating the extract are not particularly limited, while there should be employed a method and conditions which ensure that the resultant concentrate has a specific content of corosolic acid. That is, the concentrate preferably has a corosolic acid content of 0.1 to 15 mg per 100 mg of the concentrate (dry solid substance). The corosolic acid content per 100 mg of the concentrate is preferably 0.2 to 12 mg, particularly preferably 0.5 to 10 mg.

Since components other than corosolic acid contained in the concentrate of the present invention also have an effect on the activity, the other components must be taken into consideration for components to be extracted and a concentrating method and conditions. A preferred method and conditions will be understood from an explanation to be given later.

### Method 1:

In this method, a pulverized product of dry leaves of Lagerstroemia Speciosa, Linn. or Pers. (raw material) is added to ethanol or an ethanol aqueous solution (ethanol content of 50 to 80 % by weight) in an amount 5 to 20 times, preferably 8 to 10 times the weight of the raw material, and the mixture is refluxed under heat at a temperature between room temperature and 90° C, preferably approximately between 50° C and 85° C, for 30 minutes to 2 hours. The above extraction is repeated twice or three times. The resultant extract may be decolorized as required by adding activated carbon in an amount of 5 to 10 % by weight based on the raw material. Decolorization is useful for expanding the use range of the composition of the present invention. Then, the extract is filtered and concentrated at a temperature of 60°C or lower under reduced pressure to obtain a solid which is then dried at a temperature between 50°C and 70° C under reduced pressure (lower pressure than that during the concentration). The thus-obtained solid is pulverized to obtain a powdery concentrate. The concentrate obtained by the above method has a specific content of corosolic acid and contains effective amounts of other components as well.

### Method 2:

This method is an extraction method using methanol or a methanol aqueous solution. In this method, extraction is carried out in methanol or a methanol aqueous solution (methanol content of 50 to 90 % by weight) in an amount 3 to 20 times the weight of the raw material. This extraction operation is preferably carried out at a temperature between room temperature and 65°C for 30 minutes to 2 hours. The number of times of the extraction operation is not limited to one but may be 2 or more. The obtained extract is decolorized as required and concentrated under the same conditions as those in the above method 1 to obtain a solid.

### Method 3:

This method 3 is an extraction method using hot water. Extraction is carried out at a temperature between 50°C and 90°C, preferably between 60°C and 85°C, for 30 minutes to 2 hours, by using hot water in an amount 3 to 20 times the weight of the raw material. Desirably, concentration and drying after extraction are carried out for a relatively short period of time since active components may be sometimes deteriorated when the concentrate is maintained at a high temperature for a long period of time. For this reason, it is advantageous to carry out concentration and drying under reduced pressure.

The above methods 1 to 3 have been described to explain basic methods and conditions for obtaining the concentrate and may be altered and/or combined as required. For example, the method 1 and the method 2 may be combined. Out of the above methods 1 to 3, the methods 1 and 2 are preferred, and the method 1 is particularly preferred.

A concentrate containing 0.1 to 15 mg of corosolic acid per 100 mg of the concentrate is thus obtained. This concentrate may be in a powder, tablet or granular form.

The aqueous liquid composition to be vaporized of the present invention comprises the above concentrate and an aqueous medium. The aqueous medium may be water or a mixed solution of water and an alcohol. The aqueous medium may form a stable solution or dispersion and is useful for vaporizing the active ingredients thereof effectively. The amount of the concentrate in the aqueous liquid composition is determined by vaporizing means and conditions, the size of a room, vaporization time and the condition of a patient but is generally 0.5 to 3 % by weight, preferably 0.7 to 2.5 % by weight, particularly preferably 0.8 to 2.0 % by weight based on the amount of corosolic acid.

The alcohol is preferably a lower alcohol such as methanol, ethanol or propanol. They may be used in admixture. Ethanol is the most preferred. A small amount of other organic solvent may be contained, in addition to the alcohol and water. Water may be used as the solvent of the present invention and a mixed solvent of water and an alcohol may also be used. An emulsifier or dispersant may be blended as required.

When the aqueous liquid composition containing the concentrate in the above amount is prepared, it is possible to vaporize and scatter active ingredients thereof effectively and administer them from the mucous membranes of the throat and the nasal cavity through the respiratory passage.

According to the present invention, there is provided a heating vaporizer for inhibiting an increase in blood sugar level, which comprises a container for containing the above aqueous liquid composition and a porous material which is installed in the container for sucking up the aqueous liquid composition and vaporizing active ingredients thereof by heating at a top end portion thereof and which extends above the container from a bottom portion of the container.

According to the present invention, there is provided a vaporizer for an aqueous liquid composition for inhibiting an increase in blood sugar level, which comprises:
(1) a container (I) for containing an aqueous liquid composition prepared by dissolving or dispersing a concentrate of a hot water extract or alcohol extract of leaves of Lagerstroemia Speciosa, Linn. or Pers., and
(2) an ultrasonic wave generating element installed under the container (I) to nebulize the aqueous liquid composition in the container (I) into the air.

This vaporizer is called "nebulizing vaporizer".

### Brief Description of the Drawings

Fig. 1 is a sectional view of a heating vaporizer for inhibiting an increase in blood sugar level of the present invention when seen from the side;
Fig. 2 is a sectional view of the structure of Fig. 1 from which a cap is removed;
Fig. 3 is a diagram showing the sectional structure of the heating vaporizer of the present invention;
Fig. 4 is a sectional view of a nebulizing vaporizer for inhibiting an increase in blood sugar level of the present invention when seen from the side; and
Fig. 5 is a sectional view of a nebulizing vaporizer according to another embodiment of the present invention.

### Explanation of Reference Numerals

1 container
2 cap
3 porous material
4 cover
5 domed box
6 heater
7 cord
8 switch
9 opening
10 domed box
11 container (I)
12 ultrasonic wave generating element
13 exhaust port
14 container (II)

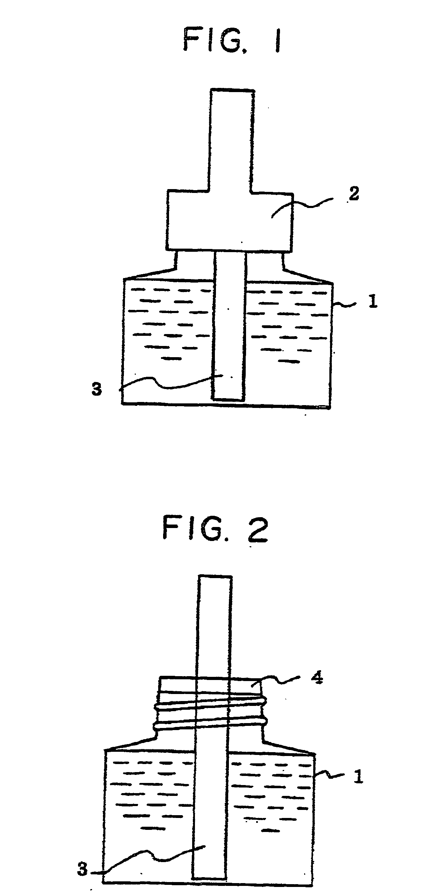

The heating vaporizer of the present invention will be first described with reference to the accompanying drawings. Fig. 1 is a sectional view of the heating vaporizer when seen from the side and Fig. 2 is a diagram of the vaporizer from which a cap 2 is removed (diagram of the structure in use).

This vaporizer is such that a container 1 is filled with a liquid composition. A porous material 3 extending above the container 1 from a bottom portion of the container 1 is installed in the container 1. This porous material 3 can suck up the liquid composition from the container 1 to a top end portion thereof according to a capillary phenomenon and the liquid composition is gradually vaporized and scattered into the air from the top end portion of the porous material 3 by heating with a heater 6 installed around the top end portion. Heating may be carried out with electricity or microwaves. Electricity is preferred.

The container 1 has such a structure that the aqueous liquid composition is gradually sucked up by the rod-shaped porous material 3 and hence, a portion around the porous material 3 is covered (or stoppered) at a top portion of the container. It is undesired that the aqueous liquid composition is vaporized or scattered to the outside of the container from a portion other than the porous material 3.

The capacity of the container for the liquid composition in the vaporizer, which is affected by its use period, is generally 15 to 100 ml, preferably 20 to 90 ml. The porous material 3 has a long diameter (diameter when its cross section is round) of 2 to 15 mm, preferably 3 to 10 mm. The porous material 3 does not need to have the same cross section in a longitudinal direction and upper and lower portions thereof may differ from each other in sectional form and area. The sectional form may be circular, oval or rectangular (regular tetragonal, rectangular parallelepiped). The length of the porous material 3 is not particularly limited but is generally 50 to 100 mm, preferably 55 to 90 mm.

The porous material 3 can suck the liquid composition up to a top end portion thereof and may have a porous structure and heat resistance to heating at the top end portion. An inorganic porous material is suitable.

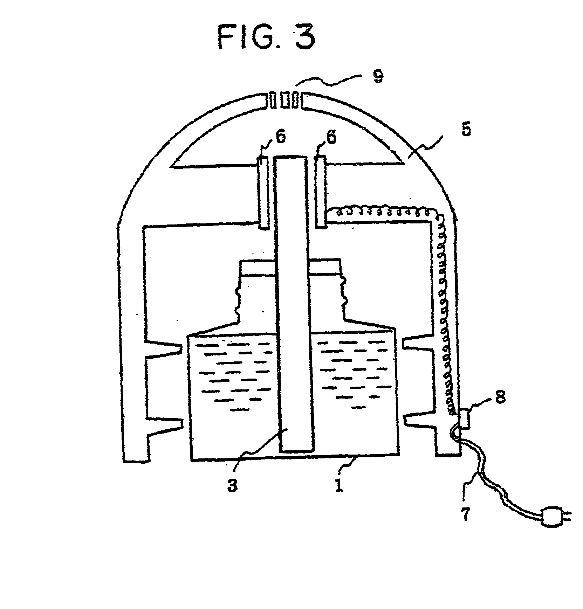

Fig. 3 is a diagram showing the sectional structure of the vaporizer of the present invention. As shown in Fig. 3, the container 1 containing the liquid composition and the porous material 3 installed therein is stored in a domed box 5. The domed box 5 has such a structure that the container 1 is stored therein and a top end portion of the porous material 3 projecting above the container 1 is surrounded by a heater 6 with a predetermined space therebetween. Preferably, the heater 6 is cylindrical when the porous material 3 has a circular sectional form. Desirably, the heater 6 and the porous material 3 are separated from each other with a predetermined space therebetween and the space therebetween is generally 0.5 to 3 mm, preferably 0.8 to 2.5 mm though it differs according to the heating temperature of the heater 6. The heater 6 is generally a heater which generates heat with electrically, preferably a ceramic heater which is safe advantageously. The heater 6 is advantageously a heater which can heat a portion corresponding to the center of the porous material 3 at 50 to 100°C, preferably 60 to 80°C when the porous material 3 is not installed. Desirably, the heater 6 can heat with electricity supplied from a domestic power source (100 to 220 V) over a cord 7 in consideration of the situation of a user (patient). The top end portion of the porous material 3 is heated by the heater 6, and active ingredients contained in the liquid composition are vaporized and scattered into the air and introduced into the room from an opening 9 formed in an upper portion of the domed box 5. Preferably, a switch 8 is provided in the vaporizer to power off the vaporizer when it is not used.

A description is subsequently given of a nebulizing vaporizer according to another embodiment of the present invention. This nebulizing vaporizer makes use of the principle of converting liquid water into fine particle using an ultrasonic wave generating element. This principle is used for humidifiers for domestic use as well.

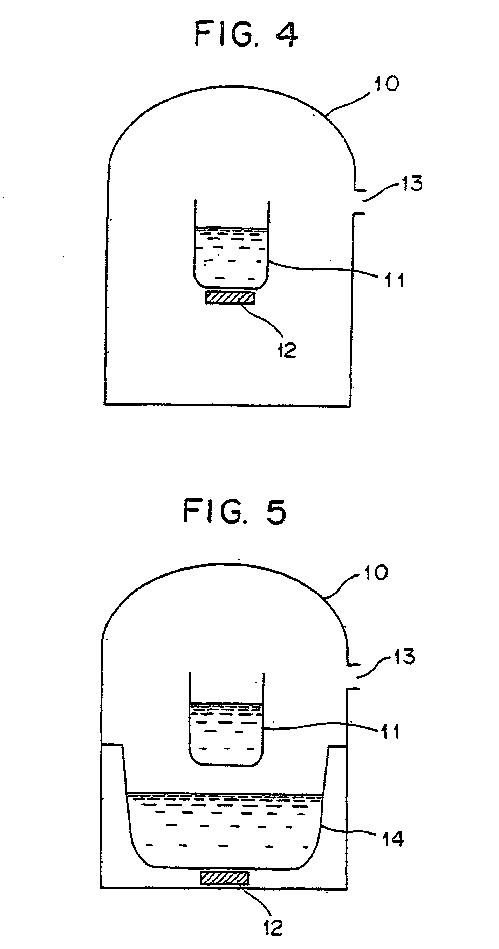

Fig. 4 and Fig. 5 are sectional views of the nebulizing vaporizer of the present invention when seen from the side. These figures show only basic members for nebulizing the aqueous liquid composition. In Fig. 4, a container (I) 11 containing the aqueous liquid composition is installed in the domed box 10. The ultrasonic wave generating element 12 is installed under the container (I) 11. Preferably, the ultrasonic wave generating element 12 is activated by a domestic power source (100 to 220 V). When the aqueous liquid composition is charged into the container (I) 11 and the ultrasonic wave generating element 12 is activated, liquid water is converted into droplets and mist which are emitted from the liquid surface. At this point, the concentrate contained in the liquid composition is scattered into the air, accompanied by the droplets. A misty product from the exhaust port 13 of the domed box 10 is scattered into the air (in the room) and the droplets are gasified. As a result, the concentrate is scattered into the air.

In the nebulizing vaporizer of Fig. 4, one container (I) 11 for the liquid composition is installed. The container (I) 11 may have such a structure that the liquid composition or water is supplied from another container not shown in Fig. 4. The installation of another container makes it possible to operate the vaporizer for a long period of time.

Fig. 5 shows a vaporizer comprising two containers. That is, the container (I) 11 filled with the liquid composition and a container (II) 14 filled with water are installed in the domed box 10.

The vaporizer of Fig. 5 nebulizes the liquid composition in the container (I) 11 and water in the container (II) 14, mixes together the two nebulized products in the domed box and discharges the mixture into the room from a single exhaust port 13. In Fig. 5, the ultrasonic wave generating element 12 is installed under the container (II) 14. As shown in Fig. 5, the container (I) 11 is installed above the container (II) 14 to nebulize the liquid composition in the container (I) 11 by the function of vibration of the ultrasonic wave generating element 12 installed under the container (II) 14. The vaporizer of Fig. 5 does not need to have two ultrasonic wave generating elements.

The vaporizer of Fig. 5 may have such a structure that the liquid composition or water is supplied into the container (I) 11 or the container (II) 14 from another unshown container.

In nebulization by the ultrasonic wave generating element, the particle size of each mist-like droplet is preferably 2 to 500 µm, more preferably 5 to 400 µm. When the size of the droplet is larger than 500 µm, the discharged droplets may not be gasified and moisten the floor or wall disadvantageously according to temperature and humidity in the room. The particle size of the droplet is preferably small for the volatization of the concentrate contained in the liquid composition.

In the nebulization of water with ultrasonic waves, the particle size of the droplet mainly depends upon the frequency of the ultrasonic waves. The frequency for generating droplets having the above particle size is approximately 1 kHz to 10 MHz, preferably approximately 5 kHz to 5 MHz. To generate small droplets, a frequency of approximately 50 kHz to about 5 MHz is advantageous.

In the case of a vaporizer making use of ultrasonic waves, the content of the concentrate in the liquid composition may be smaller than in the case of a heating vaporizer. For example, the content of the concentrate in the liquid composition is 0.01 to 0.5 % by weight, preferably 0.02 to 0.3 % by weight.

The vaporizer of the present invention is installed and used in a room where a person who is expected to suffer an increase in blood sugar level or who has a little higher blood sugar level than a healthy person (to be referred to as "quasi-patient") lives. That is, the vaporizer is installed in a living room or bed room where the quasi-patient lives to vaporize the liquid composition so that trace amounts of active ingredients are scattered into the air in the room. The quasi-patient breathes while living to absorb the active ingredients into his/her body from the mucous membranes of the throat and the nasal cavity spontaneously. While living a normal life in the room, the quasi-patient can control his/her blood sugar level to a normal level. Therefore, he/she is free from the trouble of orally taking medicine at predetermined times.

### Effect of the Invention

The liquid composition of the present invention is administered through the respiratory passage to inhibit an increase in or lowering a blood sugar level and has the following advantages.
(i) It has been reported that conventional oral preparations for the therapy of diabetes such as a sulfonyl urea preparation, a biguanide preparation and an insulin resistant amelioration preparation, etc., cause side effects such as hepatopathy, disorder of digestive organs, nausea, vomitting, etc., while the composition of the present invention is free of these side effects.
(ii) The above conventional preparations for the therapy of diabetes end their effects when the administration thereof is discontinued, while the liquid composition of the present invention continues to have an effect and has a continuing effect like traditional Chinese medicine since the blood sugar level does not increase immediately when its administration is stopped.
(iii) The liquid composition of the present invention does not cause a decrease in blood sugar level when people having a normal blood sugar level takes it.
(iv) It is considered that the above advantages of the liquid composition of the present invention are exhibited since corosolic acid contained in leaves of Lagerstroemia Speciosa, Linn. or Pers. is absorbed from the respiratory passage and activates grape sugar transportation even if it is vaporized into the air in a very low concentration.
   It is considered that intensification of the grape sugar transportation activity of corosolic acid in "intaking of sugar" an "conversion of sugar to energy" is a function different from that of conventional preparations for the therapy of diabetes.
(v) The conventional preparations for the therapy of diabetes must be orally administered several times regularly every day. The liquid composition of the present invention can be naturally administered into a living space (in a room) while living. Therefore, the blood sugar level can be reduced without making a person feel like being administered medicine. When the liquid composition is administered to a healthy person, there is no problem. In addition, it is odorless and gives no unpleasant feeling.
(vi) It is also assumed that the liquid composition of the present invention has another activity in inhibiting the digestion and absorption of glucide by preventing the function of a typical digestive enzyme for glucide. It is considered that the above activity is caused by the interaction of corosolic acid and other component(s) in the concentrated extract of leaves of Lagerstroemia Speciosa, Linn. or Pers.

### Examples

The following examples are given to further illustrate the present invention.

### Example 1

### (1) preparation of concentrate from dry leaves of Lagerstroemia Speciosa, Linn. or Pers.

1 kg of dry leaves of Lagerstroemia Speciosa, Linn. or Pers. from the Philippines were cut, placed in 5 liters of a 80 wt% ethanol aqueous solution and extracted by heating under reflux (approximately 85°C) for 1.5 hours. After extraction, the leaves of Lagerstroemia Speciosa, Linn. or Pers. were separated by filtration, again placed in a 80 wt% ethanol aqueous solution and extracted by heating under reflux (approximately 85°C) for 1.5 hours. The leaves of Lagerstroemia Speciosa, Linn. or Pers. were separated by filtration. Extracts obtained by the first and second extraction operations were combined, and 500 g of activated carbon was added to carry out decolorization. After the activated carbon was removed, ethanol and water were removed under reduced pressure at 60°C to give a concentrate. Then, the concentrate was maintained under further reduced pressure at 60° C to give a dry solid. The solid was pulverized to give 150 g of a powdery concentrate.

### (2) analysis of corosolic acid

1 g of the powdery concentrate obtained in (1) above was dissolved in 10 ml of methanol and analyzed by high-performance liquid chromatography (HPLC) to show a corosolic acid content of 30 mg in the above concentrate (corresponding to 3 mg of corosolic acid per 100 mg of the concentrate).

### (3) preparation of liquid composition

150 g of the powdery concentrate obtained in (1) above was charged into a container containing 380 g of ethanol to be dissolved in the ethanol. The obtained solution had a corosolic acid content of approximately 1.2 wt%.

### Example 2 (test on function of inhibiting an increase in blood sugar level)

The liquid composition (having a corosolic acid content of 1.2 wt%) obtained in Example 1 was vaporized and administered to a rat having a high blood sugar level by inhalation through the respiratory passage. Thirty minutes, 90 minutes and 6 hours after administration, the function of inhibiting an increase in blood sugar level was investigated. It was compared with a controlled composition (ethanol).

### (1) rats used in test

Ten 9-week old male rats (SPF/VAN Crj: Winstar) having average weight of 250 g at the time of use, purchased from Nippon Charles River Co., Ltd. were used in a test.

The rats were placed in a polycarbonate cage (265 mm (W) x 412 mm (D) x 200 mm (H)) maintained at a temperature of 23±2° C, a humidity of 50±10 %, a ventilation cycle of 15 times or more/hour and an illumination time of 12 hours/day (7:00 to 19:00) during a testing period. They were placed in the above cage in groups of two or three during a taming and quarantine period and individually for a test period.

Solid feed MF for rats (of Oriental Kobo Kogyo Co., Ltd.) and city water packed in a polycarbonate bottle were freely given to the rats.

### (2) test method

### (I) grouping and rats

A total of 10 rats in two groups were used in a test. group 1 (administered with ethanol) number of rats: 5 (rat Nos. 1 to 5)
group 2 (administered with the liquid composition) number of rats: 5 (rat Nos. 6 to 10)

### (ii) administration route and time

Administration is carried out through the respiratory passage and each group of rats are caused to inhale under a vaporized atmosphere (vaporizing amount of about 0.5 ml/hr).

### (iii) administration method

The administration method is to place each rat in a fixer and each group is placed in a test chamber having a capacity of approximately 30 m². Ethanol was vaporized in the test chamber of the first group and the composition was vaporized in the test chamber of the second group.

### (iv) preparation of rats having high blood sugar level

This was carried out by injection with streptozotocin. That is, each rat was injected with streptozotocin (to be abbreviated as STZ hereinafter) to be diseased with diabetes and observed. STZ was dissolved in a 50 mM citric acid buffer solution (pH of 4.5) to a concentration of 35 mg/ml and injected into the vein of the tail of a rat in an amount of 70 mg/kg. When the blood sugar level each of the rat was measured 5 days after injection with STZ, all the rats had a blood sugar level of 200 mg/dl or more (average of 411.5 mg/dl). Therefore, they were used in a test as STZ injected rats having a high blood sugar level.

### (v) blood sugar level measuring method

The rat was placed in a fixer, the vein of its tail was prickled with a 24G injection needle to bleed (about 0.1 ml), a serum was separated from the blood, and the absorbance of the blood was measured with a spectrophotometer (505 nm) using the glucose CII-test Wako of a mutarotase·GOD method (of Wako Junyaku Kogyo Co., Ltd.). The measurement of a blood sugar level was carried out before the administration of a test material and 30 minutes, 90 minutes and 6 hours after the administration.

### (vi) statistical processing

The obtained blood sugar levels were converted into percentage when the value of each test material before administration was 100 to obtain a change rate (%).

A significant difference test was carried out using this change rate and a Student-t test was carried out on a group of rats administered with a controlled material (ethanol) and a group of rats administered with the liquid composition. The significant level should be smaller than a critical level of 5 % (p < 0.05).

### (vii) results of change rate

The mean of change rates of Group 1 and Group 2 and SD (deviation value) were obtained. The results are shown in the table below.

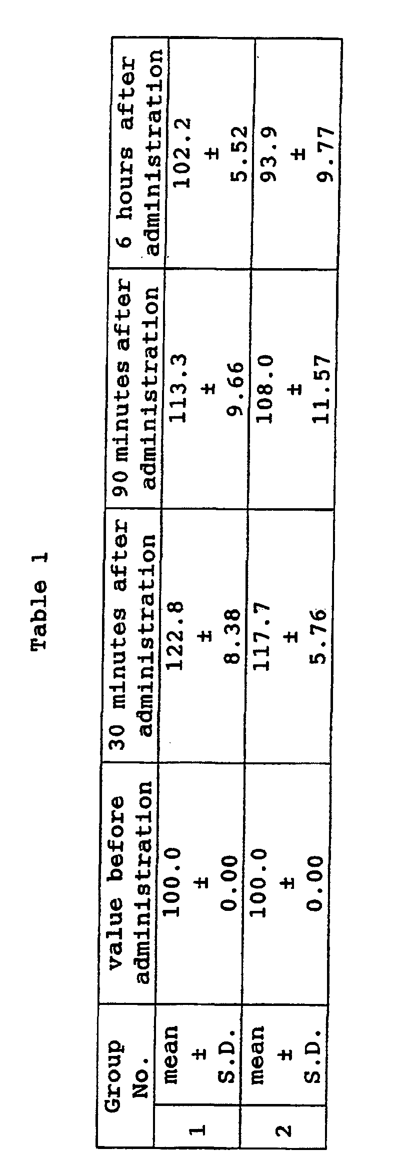

As is obvious from the above table, a significant difference with an effective level of 10 % was observed in Group 2 with respect to Group 1 about 6 hours after administration. Therefore, it was confirmed that the liquid composition of the present invention has the effect of inhibiting an increase in blood sugar level when it is vaporized and administrated through the respiratory passage.

## Claims

1. A vaporizer comprising an aqueous liquid composition in a container for inhibiting an increase in blood sugar level **characterized in that** said composition is obtainable by dissolving or dispersing a concentrate of a hot water extract or alcohol extract of leaves of Lagerstroemia Speciosa, Linn. or Pers. as an active ingredient in an aqueous medium, wherein the concentrate in the composition has a corosolic acid content of 0.5 to 3 wt%.

2. The vaporizer of claim 1, wherein the alcohol is a lower alcohol.

3. The vaporizer of claim 1 or 2 comprising:
(1) a container containing said aqueous liquid composition , and
(2) a porous material which is installed in the container for sucking up the aqueous liquid composition and vaporizing an active ingredient by heating at a top end portion thereof and which extends above the container from a bottom portion of the container.

4. The vaporizer of claim 3 further comprising:
a heater for heating a top end portion of a porous material at an upper portion of the vaporizer.

5. The vaporizer of claim 4, wherein the heater is an electric ceramic heater.

6. The vaporizer of claim 1 further comprising:
an ultrasonic wave generating element installed under the container (I) for nebulizing the aqueous liquid composition in the container (I) and scattering it into the air.

7. The vaporizer of claim 1
further comprising:
a container (II) for containing water, and
an ultrasonic wave generating element under the container (II) for nebulizing the aqueous liquid composition in the container (I) and water in the container (II) and scattering them into the air.

8. Use of
a concentrate of a hot water extract or alcohol extract of leaves of Lagerstroemia Speciosa, Linn. or Pers., wherein the concentrate in the composition has a corosolic acid content of 0.5 to 3 wt%,
for the preparation of a medicament
for inhibiting an increase in the human blood sugar level, wherein said medicament is to be administered to a subject to be treated through the respiratory passage.

9. The use of claim 8, wherein
the concentrate in an aqueous medium is to be vaporized by heating and to be scattered into the air.

10. The use of claim 8, wherein
the concentrate in an aqueous medium is to be absorbed from one end of a porous material and the other end of the porous material is to be heated to vaporize the concentrate and scatter it into the air.

11. The use of claim 8, wherein
the concentrate in an aqueous medium is to be vaporized by an ultrasonic wave generating element to scatter the concentrate into the air.

12. The use of claim 8, wherein
the concentrate in an aqueous medium is to be vaporized by an ultrasonic wave generating element, water is to be vaporized by the ultrasonic wave generating element, and the both vaporized products are to be combined to scatter the concentrate into the air.

## Patentansprüche

1. Verdampfer, der eine wässrige flüssige Zusammensetzung zur Verhinderung des Anstiegs des Blutzuckerspiegels in einem Container umfasst, **dadurch gekennzeichnet, dass** die Zusammensetzung durch Auflösen oder Dispergieren eines Konzentrats eines Heißwasserextrakts oder eines Alkoholextrakts aus Blättern von Lagerstroemia Speciosa, Linn. oder Pers., als ein aktives Ingredienz in einem wässrigen Medium erhältlich ist, wobei das Konzentrat in der Zusammensetzung einen Korosolsäuregehalt von 0,5 bis 3,0 Gew.-% besitzt.

2. Verdampfer nach Anspruch 1, wobei der Alkohol ein niederer Alkohol ist.

3. Verdampfer nach Anspruch 1 oder 2, der das Folgende umfasst:
(1) einen Behälter, der die wässrige flüssige Zusammensetzung enthält, und
(2) ein poröses Material, das in den Behälter installiert ist, um die wässrige flüssige Zusammensetzung nach oben zu saugen und ein aktives Ingredienz durch Erwärmen an einem oberen Endteil davon zu verdampfen, und das sich von einem unteren Teil des Containers über dem Container erstreckt.

4. Verdampfer nach Anspruch 3, der des Weiteren umfasst:
eine Heizvorrichtung zum Erwärmen eines oberen Endteils eines porösen Materials im oberen Teil des Verdampfers.

5. Verdampfer nach Anspruch 4, wobei die Heizvorrichtung eine elektrische Keramikheizvorrichtung ist.

6. Verdampfer nach Anspruch 1, der des Weiteren umfasst:
ein unter dem Behälter (I) installiertes Element, das Ultraschallwellen erzeugt, um die flüssige wässrige Zusammensetzung in dem Container (I) zu aerosolisieren und sie in die Luft zu zerstäuben.

7. Verdampfer nach Anspruch 1, der des Weiteren umfasst:
einen Container (2), um Wasser aufzunehmen, und
ein Ultraschallwellen-erzeugendes Element unter dem Container (II), um die flüssige Zusammensetzung in dem Container (I) und Wasser in dem Container (II) zu aerosolisieren und diese in die Luft zu zerstäuben.

8. Verwendung eines Konzentrats eines Heißwasserextrakts oder eines Alkoholextrakts von Blättern von Lagerstroemia Speciosa, Linn. oder Pers., wobei das Konzentrat in der Zusammensetzung einen Korosolsäuregehalt von 0,5 bis 3 Gew.-% besitzt, zur Herstellung eines Medikaments zur Inhibierung eines Anstiegs des menschlichen Blutzuckerspiegels, wobei das Medikament an eine zu behandelnde Person durch den Respirationstrakt verabreicht werden soll.

9. Verwendung nach Anspruch 8, wobei das Konzentrat in einem wässrigen Medium durch Erwärmen verdampft und in die Luft zerstäubt werden soll.

10. Verwendung nach Anspruch 8, wobei das Konzentrat in einem wässrigen Medium von einem Ende eines porösen Materials absorbiert werden soll und das andere Ende des porösen Materials erwärmt werden soll, um das Konzentrat zu verdampfen und es in die Luft zu zerstäuben.

11. Verwendung nach Anspruch 8, wobei das Konzentrat in einem wässrigen Medium durch ein Ultraschallwellen-erzeugendes Element verdampft werden soll, um das Konzentrat in die Luft zu zerstäuben.

12. Verwendung nach Anspruch 8, wobei das Konzentrat in einem wässrigen Medium durch ein Ultraschallwellen-erzeugendes Element verdampft werden soll, Wasser durch das Ultraschallwellen-erzeugende Element verdampft werden soll und die beiden verdampften Produkte kombiniert werden sollen, um das Konzentrat in die Luft zu zerstäuben.

## Revendications

1. Vaporisateur comportant une composition liquide aqueuse dans un conteneur destinée à inhiber une augmentation du taux de glycémie sanguine, **caractérisé en ce que** ladite composition peut être obtenue en dissolvant ou en dispersant un concentré d'un extrait d'eau chaude ou d'un extrait d'alcool de feuilles de Lagerstroemia Speciosa, Linn. ou Pers. en tant qu'ingrédient actif dans un milieu aqueux, dans lequel le concentré présent dans la composition a une teneur en acide corosolique allant de 0,5 à 3 % en poids.

2. Vaporisateur selon la revendication 1, dans lequel l'alcool est un alcool inférieur.

3. Vaporisateur selon la revendication 1 ou 2, comportant :
(1) un conteneur contenant ladite composition liquide aqueuse, et
(2) un matériau poreux qui est installé dans le conteneur pour pomper la composition liquide aqueuse et vaporiser un ingrédient actif par chauffage au niveau de sa partie d'extrémité haute et qui s'étend au-dessus du conteneur depuis une partie basse du conteneur.

4. Vaporisateur selon la revendication 3, comportant de plus :
un dispositif de chauffage destiné à chauffer une partie d'extrémité haute d'un matériau poreux au niveau d'une partie supérieure du vaporisateur.

5. Vaporisateur selon la revendication 4, dans lequel le dispositif de chauffage est un dispositif de chauffage électrique en céramique.

6. Vaporisateur selon la revendication 1, comportant de plus :
un élément de génération d'ondes ultrasonores installé sous le conteneur (I) destiné à nébuliser la composition liquide aqueuse dans le conteneur (I) et la disperser dans l'air.

7. Vaporisateur selon la revendication 1, comportant de plus :
un conteneur (II) destiné à contenir de l'eau, et
un élément de génération d'ondes ultrasonores situé sous le conteneur (II) destiné à nébuliser la composition liquide aqueuse dans le conteneur (I) et de l'eau dans le conteneur (II) et à les disperser dans l'air.

8. Utilisation d'un concentré d'un extrait d'eau chaude ou d'un extrait d'alcool de feuilles de Lagerstroemia Speciosa, Linn. ou Pers., dans laquelle le concentré présent dans la composition a une teneur en acide corosolique allant de 0,5 à 3 % en poids,
destinée à la préparation d'un médicament pour inhiber une augmentation du taux de glycémie sanguine chez l'humain,
dans laquelle ledit médicament doit être administré à un sujet devant être traité à travers la voie respiratoire.

9. Utilisation selon la revendication 8, dans laquelle le concentré présent dans un milieu aqueux doit être vaporisé par chauffage et doit être dispersé dans l'air.

10. Utilisation selon la revendication 8, dans laquelle le concentré présent dans un milieu aqueux doit être absorbé depuis une première extrémité d'un matériau poreux et l'autre extrémité du matériau poreux doit être chauffée pour vaporiser le concentré et le disperser dans l'air.

11. Utilisation selon la revendication 8, dans laquelle le concentré présent dans un milieu aqueux doit être vaporisé par un élément de génération d'ondes ultrasonores pour disperser le concentré dans l'air.

12. Utilisation selon la revendication 8, dans laquelle le concentré présent dans un milieu aqueux doit être vaporisé par un élément de génération d'ondes ultrasonores, de l'eau doit être vaporisée par l'élément de génération d'ondes ultrasonores, et les deux produits vaporisés doivent être combinés pour disperser le concentré dans l'air.
